# EUROPEAN PATENT APPLICATION

(11) **EP 4 585 306 A1**
(43) Date of publication of application: **16.07.2025**
(21) Application number: 23863145.1
(22) Date of filing: 04.09.2023
(51) Int. Cl.: B01J 23/46, B01J 29/035, C07B 61/00, C07C 5/02, C07C 9/14

(54) **METAL CATALYST AND METHOD FOR HYDROGENOLYSIS OF CYCLIC COMPOUND USING SAME**

(30) Priority: 09.09.2022 JP 2022143413
(71) Applicant: Chiyoda Corporation, Kanagawa 220-8765 (JP); NATIONAL UNIVERSITY CORPORATION YOKOHAMA NATIONAL UNIVERSITY, Yokohama-shi Kanagawa 240-8501 (JP)
(72) Inventor: KUBOTA Yoshihiro, Yokohama-shi, Kanagawa 240-8501 (JP); INAGAKI Satoshi, Yokohama-shi, Kanagawa 240-8501 (JP); ORUI Yuki, Yokohama-shi, Kanagawa 240-8501 (JP); MAEKAWA Yuki, Yokohama-shi, Kanagawa 240-8501 (JP); IMAGAWA Kenichi, Yokohama-shi, Kanagawa 220-8765 (JP); HODOSHIMA Shinya, Yokohama-shi, Kanagawa 220-8765 (JP)
(74) Representative: Gulde & Partner
(86) International application number: PCT/JP2023/032244
(87) International publication number: WO 2024/053607

(57) **Abstract**

[Problem] To perform selective hydrogenolysis of a cyclic compound that has a five-membered ring structure and that is included in a saturated cyclic compound used as a hydrogen carrier.

[Solution] A metal catalyst used in hydrogenolysis of a cyclic compound that has a five-membered ring structure and that is included in a saturated cyclic compound used as a hydrogen carrier, comprises: a silica-based carrier as a catalyst carrier; and iridium or rhodium as a metal supported on the catalyst carrier.

## Description

### [Technical Field]

The present invention relates to a metal catalyst used in hydrogenolysis of a cyclic compound having a five-membered ring structure and to a method for hydrogenolysis of the cyclic compound using the same.

### [Background Art]

An organic chemical hydride method is a conventionally known method for transporting and storing hydrogen (refer to Non-Patent Literatures 1 and 2). The organic chemical hydride method includes a hydrogenation reaction (hydrogen storage reaction), in which an aromatic compound such as toluene is reacted with hydrogen, and a dehydrogenation reaction (hydrogen releasing reaction), in which hydrogen is released from a saturated cyclic compound (hydrogenated aromatics) such as methylcyclohexane and an aromatic compound such as toluene is thereby recovered. The organic chemical hydride method allows storage and transport of the saturated cyclic compound with hydrogen absorbed therein in a liquid state at ambient temperatures and pressures, and also allows extraction of required amount of hydrogen at the site of use by dehydrogenation of the saturated cyclic compound. The saturated cyclic compound such as methylcyclohexane, and the aromatic compound such as toluene as produced in the extraction of hydrogen from the saturated cyclic compound, are used repeatedly as holders of hydrogen (hydrogen carriers).

It is known that the hydrogenation reaction and the dehydrogenation reaction in the organic chemical hydride method can produce various by-products (impurities) as well as desired products (refer to Patent Literature 1).

### [Citation List]

### [Patent Literature]

[Non-Patent Literature 1] OKADA, Yoshimi. Energy and Resources, Vol. 39, No. 3, 168 (2018)
[Non-Patent Literature 2] OKADA, Yoshimi. Tokyo High Pressure Gas Safety Institute Bulletin, July, August and September issues of 2019
[Non-Patent Literature 3] SAI, Kyoryoku. ISHII, Mika. NANBA, Tetsuya. TSUJIMURA, Taku. TANIGUCHI, Takaaki. Lecture Preprints of the 46th Petroleum-Petrochemical Symposium of JPI, 2A03 (2016)
[Patent Literature 1] JP2007-269522A

### [Summary of Invention]

### [Technical Problem]

In the above-mentioned organic chemical hydride method, repeating the hydrogenation reaction and the dehydrogenation reaction can lead to a gradual increase in percentage of impurities mixed into hydrogen carriers. It is thus conceivable that the hydrogen carriers including the impurities are distilled, for example, to remove (separate) the impurities from the hydrogen carriers.

Specific examples of possible impurities to be mixed into the hydrogen carriers are cyclic compounds having a five-membered ring structure (hereinafter referred to as five-membered ring compounds), such as cyclopentanes, that are produced in the dehydrogenation reaction (refer to Non-Patent Literature 3). Since the five-membered ring compounds have boiling points relatively close to those of the hydrogen carriers, it is not easy to separate the five-membered ring compounds from the hydrogen carriers by distillation.

After careful study, the inventors have developed a metal catalyst that allows selective hydrogenolysis of a five-membered ring compound mixed in hydrogen carriers and that consequently facilitates separation of the five-membered ring compound from the hydrogen carriers by converting the five-membered ring compound to light hydrocarbons by the hydrogenolysis.

The present invention is intended to solve the foregoing problems of the conventional techniques. An object of the present invention is to provide a metal catalyst that allows selective hydrogenolysis of a cyclic compound that has a five-membered ring structure and that is included in a saturated cyclic compound used as a hydrogen carrier, and to provide a method for hydrogenolysis of the cyclic compound using the same.

### [Solution to Problem]

An aspect of the present invention is a metal catalyst used in hydrogenolysis of a cyclic compound that has a five-membered ring structure and that is included in a saturated cyclic compound used as a hydrogen carrier. The metal catalyst includes a silica-based carrier as a catalyst carrier, and iridium or rhodium as metal supported on the catalyst carrier.

The metal catalyst according to the aspect of the invention allows selective hydrogenolysis of a cyclic compound that has a five-membered ring structure and that is included in a saturated cyclic compound used as a hydrogen carrier. That is, the metal catalyst allows effective hydrogenolysis of the cyclic compound having a five-membered ring structure, even when the cyclic compound is mixed in the hydrogen carrier, while preventing a loss (i.e., unintended chemical reaction) of the hydrogen carrier.

In the metal catalyst according to the foregoing aspect, the metal supported on the catalyst carrier is iridium, and the metal catalyst preferably includes 0.1 to 5 wt.% iridium as the metal.

Appropriate amount of iridium supported on the catalyst carrier allows stable hydrogenolysis of a cyclic compound having a five-membered ring structure.

In the metal catalyst according to the foregoing aspect, the metal supported on the catalyst carrier is rhodium, and the metal catalyst preferably includes 0.5 to 2 wt.% rhodium as the metal.

Appropriate amount of rhodium supported on the catalyst carrier allows stable hydrogenolysis of a cyclic compound having a five-membered ring structure.

In the metal catalyst according to the foregoing aspect, the silica-based carrier preferably includes amorphous silica.

Use of the silica-based carrier having a proper structure as a catalytic carrier allows stable hydrogenolysis of a cyclic compound having a five-membered ring structure.

In the metal catalyst according to the foregoing aspect, the silica-based carrier preferably includes MFI zeolite.

Use of the silica-based carrier having a proper structure as a catalytic carrier allows stable hydrogenolysis of a cyclic compound having a five-membered ring structure.

Another aspect of the present invention is a method for hydrogenolysis, using the metal catalyst of a cyclic compound that has a five-membered ring structure and that is included in the saturated cyclic compound used as a hydrogen carrier, comprising: producing an aromatic compound, and a cyclic compound that has a five-membered ring structure as an impurity, by dehydrogenation of the saturated cyclic compound; performing hydrogenation of the aromatic compound with the impurity mixed therein; and performing hydrogenolysis of mixture of the saturated cyclic compound and the impurity as produced in the hydrogenation of the aromatic compound, using the metal catalyst.

In a system based on the organic chemical hydride method, the hydrogenolysis method according to the aspect of the invention allows selective hydrogenolysis of a cyclic compound that has a five-membered ring structure and that is included in a saturated cyclic compound used as a hydrogen carrier.

In the hydrogenolysis method according to the aspect of the invention, the hydrogenolysis is preferably carried out by feeding the mixture of the saturated cyclic compound and the impurity to the metal catalyst with a catalyst layer thereof maintained at 150 °C to 300 °C together with 50 to 99.8 mol % hydrogen.

The hydrogenolysis of the mixture of the saturated cyclic compound and the impurity together with 50 to 99.8 mol % hydrogen at appropriate catalyst temperature allows stable hydrogenolysis of a cyclic compound that has a five-membered ring structure.

### [Advantageous Effects of Invention]

The above-described aspects of the invention allow selective hydrogenolysis of a cyclic compound that has a five-membered ring structure and that is included in a saturated cyclic compound used as a hydrogen carrier.

### [Brief Description of Drawings]

Fig. 1 is a block diagram showing a schematic configuration of a hydrogen storage and transport system according to an embodiment of the invention;
Figs. 2(A) and 2(B) are diagrams respectively illustrating by-products in a dehydrogenation reaction of methylcyclohexane and the relationship among boiling points thereof; and
Fig. 3 is a diagram illustrating hydrogenolysis of methylcyclopentane.

### [Description of Embodiments]

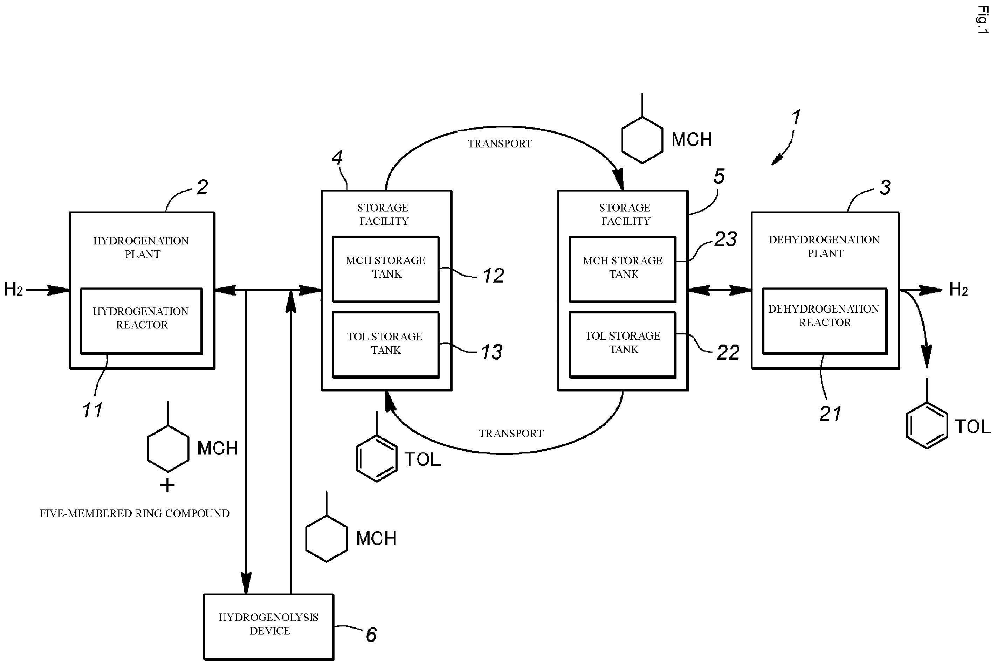

Described below with reference to the drawings are a metal catalyst according to an embodiment of the invention and a method for hydrogenolysis of a cyclic compound having a five-membered ring structure (hereinafter referred to as five-membered ring compound) using the same. Herein described is an example in which the metal catalyst and the method for hydrogenolysis using the same are applied to a hydrogen storage and transport system 1 based on the organic chemical hydride method as shown in Fig. 1.

The hydrogen storage and transport system 1 includes a hydrogenation plant 2 for producing a saturated cyclic compound as an organic hydride by adding hydrogen to an aromatic compound, and a dehydrogenation plant 3 for producing hydrogen and an aromatic compound by dehydrogenating that saturated cyclic compound. Herein described is an example in which methylcyclohexane (abbreviated as "MCH" as needed) is used as the saturated cyclic compound and toluene (abbreviated as "TOL" as needed) is used as the aromatic compound.

The hydrogenation plant 2 has a hydrogenation reaction device 11 for producing methylcyclohexane by performing a hydrogenation reaction in which to add hydrogen (H₂) to toluene based on a known method. The hydrogenation plant 2 is provided with a storage facility 4. The storage facility 4 has an MCH storage tank 12 for storing methylcyclohexane produced by the hydrogenation reaction device 11. The storage facility 4 also has a TOL storage tank 13 for storing toluene transported from a side of the dehydrogenation plant 3 (i.e., a storage facility 5).

The dehydrogenation plant 3 has a dehydrogenation reaction device 21 for producing hydrogen and toluene by performing a dehydrogenation reaction of methylcyclohexane based on a known method. The dehydrogenation plant 3 is provided with a storage facility 5. The storage facility 5 has a TOL storage tank 22 for storing toluene produced by the dehydrogenation reaction device 21. The storage facility 5 also has an MCH storage tank 23 for storing methylcyclohexane transported from a side of the hydrogenation plant 2 (i.e., the storage facility 4).

In the hydrogen storage and transport system 1, methylcyclohexane stored in the MCH storage tank 12 is transported to the dehydrogenation plant 3 using known transport means (e.g., ships, vehicles, pipelines) and stored in the MCH storage tank 23. Methylcyclohexane stored in the MCH storage tank 23 is used for a dehydrogenation reaction in the dehydrogenation reaction device 21. On the other hand, toluene stored in the TOL storage tank 22 is transported to the hydrogenation plant 2 using known transport means and stored in the TOL storage tank 13. Toluene stored in the TOL storage tank 13 is used for a hydrogenation reaction in the hydrogenation reaction device 11. Thus, methylcyclohexane and toluene are used repeatedly as holders of hydrogen (hydrogen carriers).

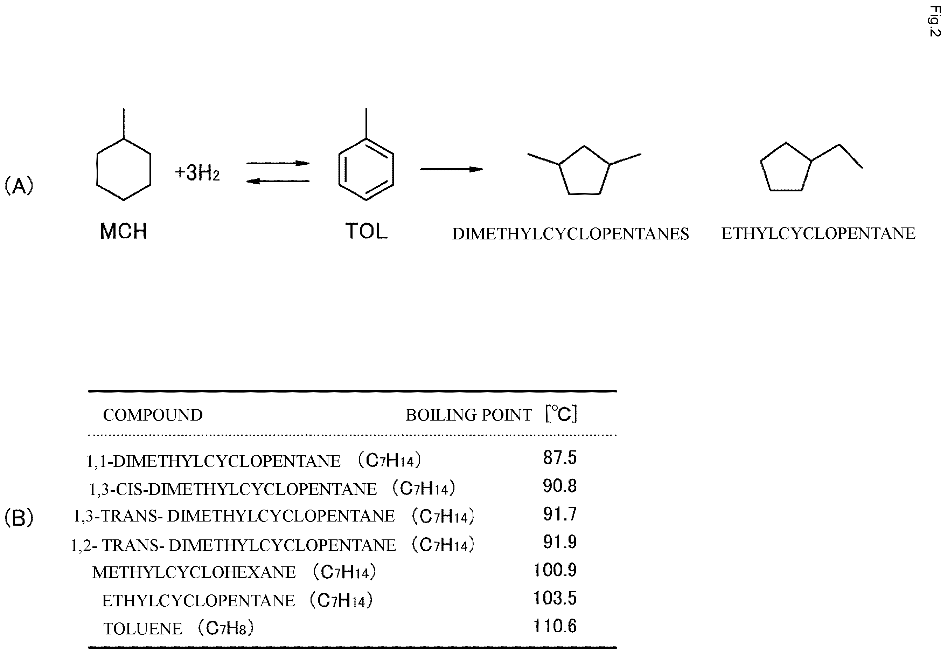

As well as hydrogen and toluene, as shown in Fig. 2(A), the dehydrogenation reaction in the dehydrogenation reaction device 21 produces by-products including five-membered ring compounds such as dimethylcyclopentanes (e.g., 1,1-dimethylcyclopentane, 1,3-cis-dimethylcyclopentane, 1,3-trans-dimethylcyclopentane, and 1,2-trans-dimethylcyclopentane) and ethylcyclopentane. On the other hand, boiling points of the five-membered ring compounds are relatively close to those of methylcyclohexane and toluene as hydrogen carriers, as shown in Fig. 2(B), and it is therefore not easy to separate the five-membered ring compounds from the hydrogen carriers by distillation.

The five-membered ring compounds as produced in the dehydrogenation reaction device 21 and mixed in toluene are stored in the TOL storage tank 22, and then sent to the hydrogenation plant 2 via the TOL storage tank 13. Methylcyclohexane produced in the hydrogenation plant 2 can thus contain as impurities the five-membered ring compounds derived from the dehydrogenation reaction as described above. The five-membered ring compounds as mixed in methylcyclohexane are sent back to the dehydrogenation plant 3 and circulated in the hydrogen storage and transportation system 1. The five-membered ring compounds can be produced not only by the dehydrogenation reaction in the dehydrogenation reaction device 21 but also by the hydrogenation reaction in the hydrogenation reaction device 11.

Therefore, the hydrogen storage and transport system 1 is provided with a hydrogenolysis device 6 for carrying out a hydrogenolysis reaction of the five-membered ring compounds mixed in the hydrogen carriers. The hydrogenolysis device 6 performs selective hydrogenolysis of the five-membered ring compounds as mixed in methylcyclohexane as impurities using a metal catalyst (hereinafter referred to as "hydrogenolysis catalyst"), thereby converting the five-membered ring compounds to light chain hydrocarbons.

The hydrogenolysis device 6 allows at least a portion of products of the hydrogenation reaction (mainly methylcyclohexane, e.g., 98 wt.% or more) produced by the hydrogenation plant 2, for example, to be extracted as processing objects before being stored in the storage facility 4 and to undergo a hydrogenolysis reaction. In the hydrogenolysis device 6, the five-membered ring compounds in the products of the hydrogenation reaction are broken down into lighter hydrocarbons (e.g., pentane, hexane, heptane, etc.). Those hydrocarbons can be removed (separated) from methylcyclohexane by distillation or other known processes. The methylcyclohexane with the five-membered ring compounds removed (or reduced) therefrom is mixed again into the products of the hydrogenation plant 2.

The processing objects of the hydrogenolysis device 6 may be methylcyclohexane (including five-membered ring compounds as impurities) extracted from the MCH storage tank 12 in the storage facility 4. In that case, the methylcyclohexane with the five-membered ring compounds removed therefrom by the hydrogenolysis device 6 is returned to the MCH storage tank 12. The hydrogenolysis device 6 may be connected to a raw material supply line (not shown) to the dehydrogenation reaction device 21 and may constitute part of the dehydrogenation plant 3.

Alternatively, the processing objects of the hydrogenolysis device 6 may be methylcyclohexane (including five-membered ring compounds as impurities) extracted from the MCH storage tank 23 in the storage facility 5. In that case, the methylcyclohexane with the five-membered ring compounds removed therefrom by the hydrogenolysis device 6 is returned to the MCH storage tank 23. The hydrogenolysis device 6 may be connected to a product discharge line (not shown) of the hydrogenation reaction device 11 and may constitute part of the hydrogenation plant 2.

The hydrogenolysis device 6 is for example a flow-type fixed-bed reaction device. The hydrogenolysis device 6 is provided with a temperature controller (e.g., an electric heater, or a heat exchanger using a heat medium). In the hydrogenolysis reaction in the hydrogenolysis device 6, the temperature of a catalyst layer consisting of a hydrogenolysis catalyst is preferably maintained within a range of 150 °C to 300 °C, for example, by the temperature controller. The metal catalyst according to the embodiment provides high catalytic performance even when the temperature of the catalyst layer is at or below 200 °C. A reaction raw material used for the hydrogenolysis reaction preferably includes, for example, 50 to 99.8 mol% hydrogen along with a mixture of methylcyclohexane and the five-membered ring compounds.

A hydrogenolysis catalyst including silica as a catalyst carrier and iridium or rhodium as a metal supported on the catalyst carrier, for example, can be used as the hydrogenolysis catalyst used in the hydrogenolysis device 6. When iridium is used, the hydrogenolysis catalyst preferably includes 0.1 to 5 wt.% iridium. More preferably, the hydrogenolysis catalyst includes 0.5 to 2 wt.% iridium. When rhodium is used, the hydrogenolysis catalyst preferably includes 0.5 to 2 wt.% rhodium. Amorphous silica or MFI zeolite is preferably used as a silica-based carrier as the catalytic carrier.

An organic hydride used in the hydrogen storage and transport system 1 may be any organic compound that at least releases hydrogen reversibly by a catalytic reaction, and is not limited to methylcyclohexane as described above. As the organic hydride, the hydrogen storage and transport system 1 can use monocyclic hydrogenated aromatic compounds such as methylcyclohexane and cyclohexane, bicyclic hydrogenated aromatic compounds such as tetralin, decalin, and methyldecalin, and tricyclic hydrogenated aromatic compounds such as tetradecahydroanthracene, singly or as a mixture of two or more of the compounds. When the organic hydride is switched from methylcyclohexane to another organic compound, toluene obtained by the dehydrogenation reaction of methylcyclohexane is replaced by another aromatic compound corresponding to the organic hydride as switched.

Preferred embodiments of the hydrogenolysis catalyst according to the invention and the method for hydrogenolysis of the five-membered ring compounds using the same are specifically described below with reference to Examples 1-7 and Comparative Example 1.

The above-described hydrogenolysis device 6 is designed to perform hydrogenolysis of the five-membered ring compounds mixed as impurities in the products of the hydrogenation reaction in the hydrogenation plant 2 (or produced as byproducts of the reaction), using the hydrogenolysis catalyst. The reaction raw material (i.e., an object of the hydrogenolysis reaction) in the hydrogenolysis device 6 is a mixture of methylcyclohexane and the five-membered ring compounds produced by the hydrogenation reaction of toluene. In Examples 1 to 6 and Comparative Example 1, methylcyclopentane (abbreviated as "MCP" as needed) was used as a model compound for the five-membered ring compounds included in (i.e., mixed in) the reaction raw material. In Example 7, ethylcyclopentane (abbreviated as "ECP" as needed) was used as a model compound for the five-membered ring compounds.

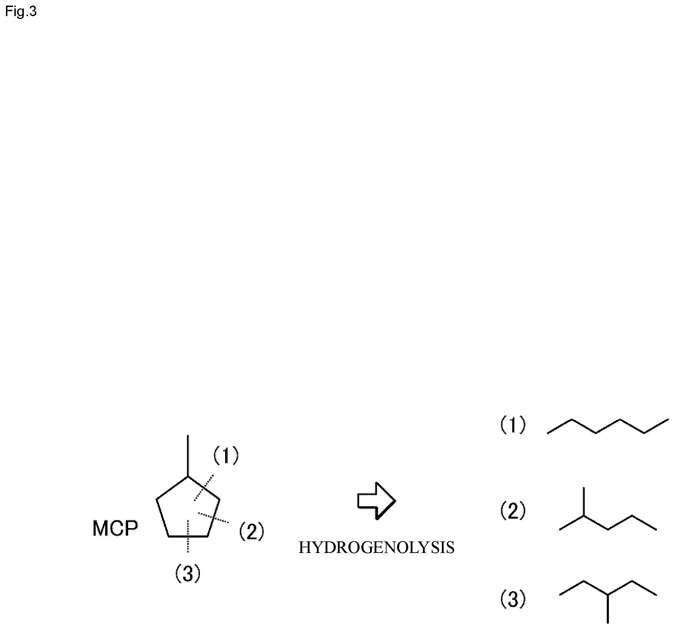

In hydrogenolysis of methylcyclopentane (MCP), for example, as shown in Fig. 3, the ring of MCP can be cleaved at positions (1) to (3) to form chain compounds according to the cleavage positions (hexane, 2-methylpentane, 3-methylpentane, etc.). Those chain compounds can be further broken down into C1 to C5 alkanes. Unlike the five-membered ring compounds, the products of the hydrogenolysis reaction can be easily separated from methylcyclohexane by distillation or other known processes. Similarly, products of hydrogenolysis of ethylcyclopentane or other five-membered ring compounds can be easily separated from methylcyclohexane.

Table 1 lists catalyst samples used in Examples 1-7 and Comparative Example 1. As will be described in detail later, Example 1 uses a catalyst with rhodium (Rh) supported on amorphous silica as a catalyst carrier. Examples 2, 4, 7, 3, and 5 use catalysts with different loadings of iridium (Ir) supported on amorphous silica. Example 6 uses a catalyst sample with iridium (Ir) supported on MFI zeolite as a catalyst carrier. Example 6 uses pure silica MFI zeolite including substantially no aluminum or other metal as heteroatoms. Comparative Example 1 uses a catalyst sample with platinum (Pt) supported on amorphous silica.

**[Table 1]**

| | Catalyst | Metal Loading [wt.%] | Carrier |
|---|---|---|---|
| Comparative Example 1 | 1.0wt%-Pt/SiO₂ | 1.0 | amorphous silica |
| Example 1 | 1.0wt%-Rh/SiO₂ | 1.0 | amorphous silica |
| Example 2.4.7 | 1.0wt%-Ir/SiO₂ | 1.0 | amorphous silica |
| Example 3 | 0.5wt%-Ir/SiO₂ | 0.5 | amorphous silica |
| Example 5 | 2.0wt%-Ir/SiO₂ | 2.0 | amorphous silica |
| Example 6 | 1.0wt%-Ir/silicalite-1 | 1.0 | MFI zeolite |

Table 2 shows reaction raw materials used in Examples 1-7 and Comparative Example 1.

**[Table 2]**

| Raw Material | MCP+H₂ (Comparative Example 1, Example 1,2) | MCH+ H₂ (Comparative Example 1, Example 1,2) | (MCP+MCH)+ H₂ (Example 3-6) | (ECP+MCH)+ H₂ (Example 7) |
|---|---|---|---|---|
| H₂/MCP Ratio[mol/mol] | 28 | - | - | - |
| H₂/MCH Ratio[mol/mol] | - | 28 | - | - |
| H₂/ECP Ratio[mol/mol] | - | - | - | - |
| H₂/(MCP+MCH) Ratio [mol/mol] | - | - | 30.3 | - |
| H₂/(ECP+MCH) Ratio [mol/mol] | - | - | - | 28 |

In Examples 1 and 2 and Comparative Example 1, performance of each catalyst sample was evaluated when reaction raw materials, methylcyclopentane (MCP) as a five-membered ring compound and methylcyclohexane (MCH) as a hydrogen carrier, were each supplied to the reaction device along with hydrogen. When MCP is used as the reaction raw material in Examples 1 and 2 and Comparative Example 1, hydrogen/MCP ratio (mol/mol) in the reaction raw material is 28, as shown in Table 2. When MCH is used as the reaction raw material, hydrogen/MCH ratio (mol/mol) in the reaction raw material is 28.

In Examples 3-6, a raw material mixture of 10 mol% methylcyclopentane as a five-membered ring compound and 90 mol% methylcyclohexane as a hydrogen carrier was prepared according to an estimate of actual raw materials to be used in the hydrogenolysis device, and the performance of each catalyst sample was evaluated when the raw material mixture was fed to the reaction device along with hydrogen. In Examples 3-6, hydrogen/(MCP+MCH) ratio (mol/mol) in the reaction raw material is 30.3, as shown in Table 2.

In Example 7, a raw material mixture of 10 mol% ethylcyclopentane (ECP) as a five-membered ring compound and 90 mol% methylcyclohexane (MCH) as a hydrogen carrier was prepared, and the performance of the catalyst sample was evaluated when the raw material mixture was fed to the reaction device along with hydrogen. In Example 7, hydrogen/(ECP+MCH) ratio (mol/mol) in the reaction raw material is 28, as shown in Table 2.

In Examples 1-7, iridium (Ir) and rhodium (Rh), which in the presence of hydrogen promote the hydrogenolysis reaction selectively to cleave carbon-carbon bonds in five-membered ring compounds, were used as active species on the assumption of the hydrogenolysis reaction being carried out in a mild temperature range where temperature of the catalyst layer is around 200 °C. For catalytst carriers, silica-based materials were used that do not show solid acid properties that may cause side reactions such as isomerization.

### [Example 1]

### Preparation method of 1.0 wt.%-Rh/SiO₂ catalyst

In a 300-mL eggplant flask, 0.0767 g of rhodium chloride trihydrate (RhCl₃·3H₂O, including 39.1 wt.% Rh) was thoroughly dissolved in 150 mL of pure water by ultrasonic irradiation, and 3.0 g of amorphous silica powder (AEROSIL (registered trademark) 380, NIPPON AEROSIL CO., LTD., BET specific surface area 380 m₂/g) as a carrier was added to the solution. The mixture was stirred at room temperature for one hour, and the silica powder was impregnated with the solution in such a manner as to have a Rh metal loading of 1 wt.%. The mixture was evaporated to dryness at 40 °C using an evaporator. The recovered product was transferred to an oven and dried at 80 °C overnight, and the resulting powder was recovered. The powder was pressed, then moderately ground, and sieved to 500-600 µm. In a catalyst reduction process, approximately 1.0 g of the catalyst as sieved was loaded into a flow-type fixed-bed reaction device, and heated up to 400 °C over the course of 30 minutes under nitrogen flow (30 mL/min). Then, the catalyst was subjected to the reduction process for five hours under hydrogen flow (50 mL/min), and cooled to room temperature with the hydrogen atmosphere maintained. Thus, the 1.0 wt.%-Rh/SiO₂ catalyst was obtained.

### Method for performance evaluation test of 1.0 wt.%-Rh/SiO₂ catalyst

### [Hydrogenolysis reaction of methylcyclopentane (MCP)]

A performance evaluation test of the 1.0 wt.%-Rh/SiO₂ catalyst as described above was conducted according to the following procedure. For pretreatment, 100.0 mg of the 1.0 wt.%-Rh/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCP was carried out for a predetermined time under the following conditions: hydrogen flow rate of 40 mL/min, H₂/MCP = 28 mol/mol, W/F (ratio of catalyst amount to MCP flow rate) = 28.5 g - cat. h/mol, reaction temperature of 200 °C, and total pressure of 0.1 MPa. Five minutes after the start of the hydrogenolysis reaction, the reaction products were sampled, and the MCP conversion (%) and the product selectivity (C-%) were determined by FID-GC (gas chromatograph with flame ionization detector) analysis as indexes of catalytic performance (for the results, refer to Table 3 to be discussed later).

### [Hydrogenolysis reaction of methylcyclohexane (MCH)]

A performance evaluation test of the 1.0 wt.%-Rh/SiO₂ catalyst as described above was conducted according to the following procedure. For pretreatment, 100.0 mg of the 1.0 wt.%-Rh/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCH was carried out for a predetermined time under the following conditions: hydrogen flow rate of 40 mL/min, H₂/MCH = 28 mol/mol, W/F (ratio of catalyst amount to MCH flow rate) = 28.5 g - cat. h/mol, reaction temperature of 200 °C, and total pressure of 0.1 MPa. Five minutes after the start of the hydrogenolysis reaction, the reaction products were sampled, and the MCH conversion (%) and the product selectivity (C-%) were determined by FID-GC analysis as indexes of catalytic performance (for the results, refer to Table 3 to be discussed later).

### [Example 2]

### Preparation method of 1.0 wt.%-Ir/SiO₂ catalyst

In a 300-mL eggplant flask, 0.0604 g of iridium chloride n-hydrate (IrCl₃·nH₂O, including 49.7 wt.% Ir) was thoroughly dissolved in 150 mL of pure water by ultrasonic irradiation, and 3.0 g of amorphous silica powder (AEROSIL 380, NIPPON AEROSIL CO., LTD., BET specific surface area 380 m₂/g) as a carrier was added to the solution. The mixture was stirred at room temperature for one hour, and the silica powder was impregnated with the solution in such a manner as to have an Ir metal loading of 1 wt.%. The mixture was evaporated to dryness at 40 °C using an evaporator. The recovered product was transferred to an oven and dried at 80 °C overnight, and the resulting powder was recovered. The powder was pressed, then moderately ground, and sieved to 500-600 µm. In a catalyst reduction process, approximately 1.0 g of the catalyst as sieved was loaded into a flow-type fixed-bed reaction device, heated up to 400 °C over the course of 30 minutes under nitrogen flow (30 mL/min), subjected to the reduction process for five hours under hydrogen flow (50 mL/min), and cooled to room temperature with the hydrogen atmosphere maintained. Thus, the 1.0 wt.%-Ir/SiO₂ catalyst was obtained.

### Method for performance evaluation test of 1.0 wt.%-Ir/SiO₂ catalyst

### [Hydrogenolysis reaction of methylcyclopentane (MCP)]

A performance evaluation test of the 1.0 wt.%-Ir/SiO₂ catalyst as described above was conducted according to the following procedure. For pretreatment, 100.0 mg of the 1.0 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCP was carried out for a predetermined time under the following conditions: hydrogen flow rate of 40 mL/min, H₂/MCP = 28 mol/mol, W/F (ratio of catalyst amount to MCP flow rate) = 28.5 g - cat. h/mol, reaction temperature of 200 °C, and total pressure of 0.1 MPa. Five minutes after the start of the hydrogenolysis reaction, the reaction products were sampled, and the MCP conversion (%) and the product selectivity (C-%) were determined by FID-GC analysis as indexes of catalytic performance (for the results, refer to Table 3 to be discussed later).

### [Hydrogenolysis reaction of methylcyclohexane (MCH)]

A performance evaluation test of the 1.0 wt.%-Ir/SiO₂ catalyst as prepared by the above-described procedure was conducted according to the following procedure. For pretreatment, 100.0 mg of the 1.0 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCH was carried out for a predetermined time under the following conditions: hydrogen flow rate of 40 mL/min, H₂/MCH = 28 mol/mol, W/F (ratio of catalyst amount to MCH flow rate) = 28.5 g - cat. h/mol, reaction temperature of 200 °C, and total pressure of 0.1 MPa. Five minutes after the start of the hydrogenolysis reaction, the reaction products were sampled, and the MCH conversion (%) and the product selectivity (C-%) were determined by FID-GC analysis as indexes of catalytic performance.

### [Example 3]

### Preparation method of 0.5 wt.%-Ir/SiO₂ catalyst

In a 300-mL eggplant flask, 0.0302 g of iridium chloride n-hydrate (IrCl₃·nH₂O, including 49.7 wt.% Ir) was thoroughly dissolved in 150 mL of pure water by ultrasonic irradiation, and 3.0 g of amorphous silica powder (AEROSIL 380, NIPPON AEROSIL CO., LTD., BET specific surface area 380 m₂/g) as a carrier was added to the solution. The mixture was stirred at room temperature for one hour, and the silica powder was impregnated with the solution in such a manner as to have an Ir metal loading of 0.5 wt.%. The mixture was evaporated to dryness at 40 °C using an evaporator. The recovered product was transferred to an oven and dried at 80 °C overnight, and the resulting powder was recovered. The powder was pressed, then moderately ground, and sieved to 500-600 µm. In a catalyst reduction process, approximately 1.0 g of the catalyst as sieved was loaded into a flow-type fixed-bed reaction device, heated up to 400 °C over the course of 30 minutes under nitrogen flow (30 mL/min), subjected to the reduction process for five hours under hydrogen flow (50 mL/min), and cooled to room temperature with the hydrogen atmosphere maintained. Thus, the 0.5 wt.%-Ir/SiO₂ catalyst was obtained.

### Method for performance evaluation test of 0.5 wt.%-Ir/SiO₂ catalyst

### [Hydrogenolysis reaction of raw material mixture of methylcyclopentane (MCP) and methylcyclohexane (MCH)]

A performance evaluation test of the 0.5 wt.%-Ir/SiO₂ catalyst as described above was conducted according to the following procedure. For pretreatment, 201.0 mg of the 0.5 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of a raw material mixture of MCP and MCH was carried out for a predetermined time under the following conditions: hydrogen flow rate of 40 mL/min, H₂/(MCP + MCH) = 30.3 mol/mol (H₂:MCP:MCH = 30.3:0.1:0.9), W/F (ratio of catalyst amount to (MCP + MCH) flow rate) = 62.1 g - cat. h/mol, reaction temperature of 200 °C, and total pressure of 0.1 MPa. Five minutes after the start of the hydrogenolysis reaction, the reaction products were sampled, and the MCP conversion (%), the MCH conversion (%), and the product selectivity (C-%) were determined by FID-GC analysis as indexes of catalytic performance (for the results, refer to Table 4 to be discussed later).

### [Example 4]

### Preparation method of 1.0 wt.%-Ir/SiO₂ catalyst

A 1.0 wt.%-Ir/SiO₂ catalyst was obtained by a similar procedure to that for the catalyst in Example 2.

### Method for performance evaluation test of 1.0 wt.%-Ir/SiO₂ catalyst

### [Hydrogenolysis reaction of raw material mixture of methylcyclopentane (MCP) and methylcyclohexane (MCH)]

A performance evaluation test of the 1.0 wt.%-Ir/SiO₂ catalyst as described above was conducted according to the following procedure. For pretreatment, 202.0 mg of the 1.0 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of a raw material mixture of MCP and MCH was carried out for a predetermined time under the following conditions: hydrogen flow rate of 40 mL/min, H₂/(MCP + MCH) = 30.3 mol/mol (H₂:MCP:MCH = 30.3:0.1:0.9), W/F (ratio of catalyst amount to (MCP + MCH) flow rate) = 62.4 g - cat. h/mol, reaction temperature of 200 °C, and total pressure of 0.1 MPa. Five minutes after the start of the hydrogenolysis reaction, the reaction products were sampled, and the MCP conversion (%), the MCH conversion (%), and the product selectivity (C-%) were determined by FID-GC analysis as indexes of catalytic performance (for the results, refer to Table 4 to be discussed later).

### [Example 5]

### Preparation method of 2.0 wt.%-Ir/SiO₂ catalyst

In a 300-mL eggplant flask, 0.1208 g of iridium chloride n-hydrate (IrCl₃·nH₂O, including 49.7 wt.% Ir) was thoroughly dissolved in 150 mL of pure water by ultrasonic irradiation, and 3.0 g of amorphous silica powder (AEROSIL 380, NIPPON AEROSIL CO., LTD., BET specific surface area 380 m₂/g) as a carrier was added to the solution. The mixture was stirred at room temperature for one hour, and the silica powder was impregnated with the solution in such a manner as to have an Ir metal loading of 0.5 wt.%. The mixture was evaporated to dryness at 40 °C using an evaporator. The recovered product was transferred to an oven and dried at 80 °C overnight, and the resulting powder was recovered. The powder was pressed, then moderately ground, and sieved to 500-600 µm. In a catalyst reduction process, approximately 1.0 g of the catalyst as sieved was loaded into a flow-type fixed-bed reaction device, heated up to 400 °C over the course of 30 minutes under nitrogen flow (30 mL/min), subjected to the reduction process for five hours under hydrogen flow (50 mL/min), and cooled to room temperature with the hydrogen atmosphere maintained. Thus, the 2.0 wt.%-Ir/SiO₂ catalyst was obtained.

### Method for performance evaluation test of 2.0 wt.%-Ir/SiO₂ catalyst

### [Hydrogenolysis reaction of raw material mixture of methylcyclopentane (MCP) and methylcyclohexane (MCH)]

A performance evaluation test of the 2.0 wt.%-Ir/SiO₂ catalyst as described above was conducted according to the following procedure. For pretreatment, 204.0 mg of the 1.0 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of a raw material mixture of MCP and MCH was carried out for a predetermined time under the following conditions: hydrogen flow rate of 40 mL/min, H₂/(MCP + MCH) = 30.3 mol/mol (H₂:MCP:MCH = 30.3:0.1:0.9), W/F (ratio of catalyst amount to (MCP + MCH) flow rate) = 63.0 g - cat. h/mol, reaction temperature of 200 °C, and total pressure of 0.1 MPa. Five minutes after the start of the hydrogenolysis reaction, the reaction products were sampled, and the MCP conversion (%), the MCH conversion (%), and the product selectivity (C-%) were determined by FID-GC analysis as indexes of catalytic performance (for the results, refer to Table 4 to be discussed later).

### [Example 6]

### Preparation method of 1.0 wt.%-Ir/silicalite-1 catalyst

Prepared was a catalyst with 1.0 wt.% Ir supported on crystalline silicalite-1 (1.0 wt.%-Ir/silicalite-1). Silicalite-1 as a carrier was synthesized by the following procedure. In an 80-mL perfluoroalkoxy alkane (PFA) container, predetermined amounts of tetrapropylammonium hydroxide (TPAOH) and fumed silica (Cab-O-Sil M5) were stirred in oil bath at 80 °C for two hours, and a base gel solution was obtained. The molar composition of the base gel solution was SiO₂:TPAOH:H₂O = 1.0:0.24:10.44. The gel solution was subjected to hydrothermal synthesis in a 23 mL autoclave under self-pressure at 135 °C for three hours at 20 rpm. Then, the resulting product was recovered by centrifugation, dried in an oven at 80 °C, calcined in a muffle furnace at 550 °C, and silicalite-1 was thus synthesized. XRD analysis confirmed that the sample as synthesized has an MFI structure, and analysis of nitrogen adsorption isotherm confirmed that the sample has a BET specific surface area of 371 m²/g and a micropore volume of 0.154 cm³/g.

A 1.0 wt.%-Ir/silicalite-1 catalyst was prepared using silicalite-1 as synthesized by the above-described procedure. In a 200-mL eggplant flask, 0.0302 g of iridium chloride n-hydrate (IrCl₃·nH₂O, including 49.7 wt.% Ir) was thoroughly dissolved in 75 mL of pure water by ultrasonic irradiation, and 1.5 g of silicalite-1 powder was added to the solution. The mixture was stirred at room temperature for one hour, and the silicalite-1 powder was impregnated with the solution in such a manner as to have an Ir metal loading of 1.0 wt.%. The mixture was evaporated to dryness at 40 °C using an evaporator. The recovered product was transferred to an oven and dried at 80 °C overnight, and the resulting powder was recovered. The powder was pressed, then moderately ground, and sieved to 500-600 µm. In a catalyst reduction process, approximately 1.0 g of the catalyst as sieved was loaded into a flow-type fixed-bed reaction device, heated up to 400 °C over the course of 30 minutes under nitrogen flow (30 mL/min), subjected to the reduction process for five hours under hydrogen flow (50 mL/min), and cooled to room temperature with the hydrogen atmosphere maintained. Thus, the 1.0 wt.%-Ir/silicalite-1 catalyst was obtained.

### Method for performance evaluation test of 1.0 wt.%-Ir/silicalite-1 catalyst

### [Hydrogenolysis reaction of raw material mixture of methylcyclopentane (MCP) and methylcyclohexane (MCH)]

A performance evaluation test of the 1.0 wt.%-Ir/silicalite-1 catalyst as described above was conducted according to the following procedure. For pretreatment, 202.0 mg of the 1.0 wt.%-Ir/silicalite-1 catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of a raw material mixture of MCP and MCH was carried out for a predetermined time under the following conditions: hydrogen flow rate of 40 mL/min, H₂/(MCP + MCH) = 30.3 mol/mol (H₂:MCP:MCH = 30.3:0.1:0.9), W/F (ratio of catalyst amount to (MCP + MCH) flow rate) = 62.4 g - cat. h/mol, reaction temperature of 200 °C, and total pressure of 0.1 MPa. Five minutes after the start of the hydrogenolysis reaction, the reaction products were sampled, and the MCP conversion (%), the MCH conversion (%), and the product selectivity (C-%) were determined by FID-GC analysis as indexes of catalytic performance (for the results, refer to Table 3 to be discussed later).

### [Example 7]

### Preparation method of 1.0 wt.%-Ir/SiO₂ catalyst

A 1.0 wt.%-Ir/SiO₂ catalyst was obtained by a similar procedure to that for the catalyst in Example 2.

### Method for performance evaluation test of 1.0 wt.%-Ir/SiO₂ catalyst

### [Hydrogenolysis reaction of raw material mixture of ethylcyclopentane (ECP) and methylcyclohexane (MCH)]

A performance evaluation test of the 1.0 wt.%-Ir/SiO₂ catalyst as described above was conducted according to the following procedure. For pretreatment, 202.0 mg of the 1.0 wt.%-Ir/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of a raw material mixture of ECP and MCH was carried out for a predetermined time under the following conditions: hydrogen flow rate of 40 mL/min, H₂/(ECP + MCH) = 28 mol/mol (H₂:MCP:MCH = 28.0:0.1:0.9), W/F (ratio of catalyst amount to (ECP + MCH) flow rate) = 57.6 g - cat. h/mol, reaction temperature of 200 °C, and total pressure of 0.1 MPa. Five minutes after the start of the hydrogenolysis reaction, the reaction products were sampled, and the ECP conversion (%), the MCH conversion (%), and the product selectivity (C-mol%) were determined by FID-GC analysis as indexes of catalytic performance (for the results, refer to Table 5 to be discussed later).

### [Comparison Example 1]

### Preparation method of 1.0 wt.%-Pt/SiO₂ catalyst

A commercial product (Aldrich, 520691-25G) for a silica-supported Pt catalyst (1.0 wt.%-Pt/SiO₂) was used as a catalyst sample.

### Method for performance evaluation test of 1.0 wt.%-Pt/SiO₂ catalyst

### [Hydrogenolysis reaction of methylcyclopentane (MCP)]

A performance evaluation test of the commercially available 1.0 wt.%-Pt/SiO₂ catalyst as described above was conducted according to the following procedure. For pretreatment, 202.0 mg of the 1.0 wt.%-Pt/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCP was carried out for a predetermined time under the following conditions: hydrogen flow rate of 40 mL/min, H₂/MCP = 28 mol/mol, W/F (ratio of catalyst amount to MCP flow rate) = 28.5 g - cat. h/mol, reaction temperature of 200 °C, and total pressure of 0.1 MPa. Five minutes after the start of the hydrogenolysis reaction, the reaction products were sampled, and the MCP conversion (%) and the product selectivity (C-%) were determined by FID-GC analysis as indexes of catalytic performance (for the results, refer to Table 3 to be discussed later).

### [Hydrogenolysis reaction of methylcyclohexane (MCH)]

A performance evaluation test of the commercially available 1.0 wt.%-Pt/SiO₂ catalyst as described above was conducted according to the following procedure. For pretreatment, 100.0 mg of the 1.0 wt.%-Pt/SiO₂ catalyst was loaded in a flow-type fixed-bed reaction device, heated up to 400 °C over the course of 30 minutes under helium flow (30 mL/min), and then subjected to a reduction process for one hour under hydrogen flow (50 mL/min). After the reduction process, a hydrogenolysis reaction of MCH was carried out for a predetermined time under the following conditions: hydrogen flow rate of 40 mL/min, H₂/MCH = 28 mol/mol, W/F (ratio of catalyst amount to MCH flow rate) = 28.5 g - cat. h/mol, reaction temperature of 200 °C, and total pressure of 0.1 MPa. Five minutes after the start of the hydrogenolysis reaction, the reaction products were sampled, and the MCH conversion (%) and the product selectivity (C-%) were determined by FID-GC analysis as indexes of catalytic performance (for the results, refer to Table 3 to be discussed later).

Table 3 shows the performance test results of each catalyst in Examples 1 and 2 and Comparative Example 1. In the performance test of each catalyst, the hydrogenolysis reactions were carried out under the same conditions with methylcyclopentane (MCP) and methylcyclohexane (MCH) respectively used as the sole reaction raw material.

**[Table 3]**

| | **Catalyst** | **Reaction Temperature [°C]** | **Raw Material (MCP + H₂) (H₂/MCP = 28 [mol/mol]** | | **Raw Material (MCH + H₂) (H2/MCH = 28 [mol/mol])** | |
|---|---|---|---|---|---|---|
| | | | **MCP Conversion[%]** | **Selectivity[C-%] n-C₆ : 3-MP : 2-MP** | **MCH Conversion[%]** | **Selectivity to TOL[C-%]** |
| **Comparative Example 1** | 1.0 wt%-Pt/SiO₂ | 200°C | < 1 | 81 : 5 : 14 | <1 | 100 |
| **Example 1** | 1.0 wt%-Rh/SiO₂ | 200°C | 53 | 7 : 29 : 64 | <1 | 100 |
| **Example** 2 | 1.0 wt%-Ir/SiO₂ | 200°C | 70 | 3 : 29 : 68 | <1 | 100 |

In Comparative Example 1 (1.0 wt.%-PT/SiO₂ catalyst), as shown in Table 3, MCP used as the raw material caused few hydrogenolysis reaction, and the MCP conversion was less than 1 %. When MCH was used as the raw material, the MCH conversion was also less than 1 %, and all the products were toluene (TOL).

Thus, platinum used as the active species supported on silica exhibited activity in the dehydrogenative aromatization reaction of cyclohexanes (herein methylcyclohexane), but very low activity in the hydrogenolysis reaction of cyclopentanes (herein methylcyclopentane).

On the other hand, the 1.0 wt.%-Rh/SiO₂ catalyst of Example 1 exhibited high activity in the hydrogenolysis reaction of MCP, and the MCP conversion was 53 %. The products obtained by the hydrogenolysis of MCP were n-hexane (n-C₆H₁₄ (see "n-C₆" in the table), 3-methylpentane (3-CH₃-C₅H₁₁ (see "3-MP" in the table)), and 2-methylpentane (2-CH₃-C₅H₁₁ (see "2-MP" in the table)) as direct ring-opening products. MCH used as the raw material caused few hydrogenolysis reaction, the MCH conversion was less than 1%, and the only product obtained was toluene (TOL).

The 1.0 wt%-Ir/SiO₂ catalyst of Example 2 exhibited improved activity in the hydrogenolysis reaction of methylcyclopentane compared to that in Example 1, and the methylcyclopentane conversion reached 70%. As in Example 1, the products obtained by the hydrogenolysis of methylcyclopentane were n-hexane, 3-methylpentane, and 2-methylpentane, as direct ring-opening products. Methylcyclohexane (MCH) used as the raw material caused few hydrogenolysis reaction, the raw material conversion was less than 1%, and the only product obtained was toluene.

Thus, it was found that rhodium or iridium as the active species supported on amorphous silica exhibits high activity in the hydrogenolysis reaction of cyclopentanes but low activity in the hydrogenolysis reaction of cyclopentanes.

Table 4 shows the performance test results of the catalysts in Examples 3 to 6. In the performance tests, the hydrogenolysis reactions were carried out under the same conditions using a raw material mixture of methylcyclopentane (MCP) and methylcyclohexane (MCH).

**[Table 4]**

| | **Catalyst** | **Raw Material : (MCP + MCH) + H₂ (H₂/(MCP + MCH) = 30.3 [mol/mol], H₂ : MCP : MCH = 30.3 : 0.1 : 0.9)** | | | | | | | | | | |
|---|---|---|---|---|---|---|---|---|---|---|---|---|
| | | **MCP Conversion[%]** | **Selectivity[C-%]** | | | **MCH Conversion** | | | **Selectivity[C-%]** | | | |
| | | | **C₁-C₅ :** | **3-MP :** | **2-MP:** | **n-C₆** | **[%]** | **C₁-C₆ :** | **3-MH :** | **2-MH :** | **n-C₇:** | **TOL** |
| **Example 3** | **0.5 wt%-Ir/SiO₂** | 8.7 | 5.0 | 31.2 | 57.0 | 6.8 | 0.3 | 0.0 | 16.7 | 12.5 | 29.5 | 41.3 |
| **Example 4** | **1.0 wt%-Ir/SiO₂** | 28.1 | 8.8 | 30.7 | 58.2 | 2.3 | 1.1 | 0.0 | 49.8 | 32.9 | 7.4 | 9.9 |
| **Example 5** | **2.0 wt%-Ir/SiO₂** | 51.3 | 11.9 | 30.5 | 56.0 | 1.6 | 5.1 | 0.0 | 52.2 | 35.0 | 8.8 | 4.0 |
| **Example 6** | **1.0 wt%-Ir/ silicalite-1** | 9.3 | 3.8 | 27.5 | 63.5 | 5.2 | 0.1 | 0.0 | 38.7 | 25.6 | 3.7 | 32.0 |

In Examples 3 to 5 (X wt.%-Ir/SiO₂ catalyst, X = 0.5, 1.0, 2.0), samples with iridium metal loadings on amorphous silica carriers ranging from 0.5 to 2.0 wt.% were used for performance evaluation.

For the catalysts in Examples 3 to 5, as shown in Table 4, the conversion of methylcyclopentane (MCP) ranged from 8.7 to 51.3 %, and the conversion of methylcyclohexane (MCH) ranged from 0.3 to 5.1 %. Thus, the conversions both became higher as the metal loadings increased. It was also confirmed that all the catalysts in Examples 3 to 5 allow highly selective hydrogenolysis of MCP while suppressing the hydrogenolysis of methylcyclohexane (MCH). In terms of product distribution, the reaction products derived from MCP were n-hexane (n-C₆H₁₄ (see "n-C₆" in the table)), 3-methylpentane (3-CH₃-C₅H₁₁ (see "3-MP" in the table)), and 2-methylpentane (2-CH₃-C₅H₁₁ (see "2-MP" in the table)), as direct ring-opening products, and light hydrocarbons (C₁ to C₅) as decomposition products. The reaction products derived from MCH were n-heptane (n-C₇H₁₆ (see "n-C7" in the table)), 3-methylhexane (3-CH₃-C₆H₁₃ (see "3-MH" in the table)), 2-methylhexane (2 -CH₃-C₆H₁₃ (see "2-MH" in the table)), as direct ring-opening products, and toluene (TOL) as a dehydrogenation product.

With regard to the 1.0 wt.%-Ir/silicalite-1 catalyst in Example 6, a sample with 1.0 wt.% iridium metal supported on silicalite-1 as a kind of MFI zeolites was used for performance evaluation.

In the catalyst of Example 6, as shown in Table 4, the MCP conversion was lower than that in Example 4 (1.0 wt.%-Ir/SiO₂ catalyst), but the MCH conversion was kept very low at 0.1 %, indicating that the catalyst is highly selective. The product distribution showed the same pattern as those in Examples 3 to 5 as described above. In Example 6, an iridium-supported catalyst was used, but the similar effect can be expected when a rhodium-supported catalyst is used.

Table 5 shows the performance test result of the catalyst in Example 7. In the performance test, the hydrogenolysis reaction was carried out using a raw material mixture of ethylcyclopentane (ECP) and methylcyclohexane (MCH).

**[Table 5]**

| | **Catalyst** | **Raw Material : (ECP + MCH) +H₂ (H₂/(ECP + MCH) = 28 [mol/mol], H₂ : ECP : MCH = 28 : 0.1 : 0.9)** | | | | | | | | | |
|---|---|---|---|---|---|---|---|---|---|---|---|
| | | **ECP Conversion** | **MCH Conversion** | | | **Selectivity[C-%]** | | | | | |
| | | **[%]** | **[%]** | **2-MH:** | **3-MH :** | **3-EP :** | **n-C₇ :** | **C₁-C₅ :** | **2-MP :** | **3-MP :** | **TOL** |
| **Example 7** | **1.0 wt%-Ir/SiO₂** | 39.8 | 2.8 | 5.2 | 55.7 | 28.3 | 2.3 | 4.4 | 1.6 | 2.4 | 4.3 |

For the catalyst in Example 7, as shown in Table 5, the ECP conversion was 39.8 %, and the MCH conversion was 2.8 %. Thus, it was confirmed that the catalyst allows highly selective hydrogenolysis of ECP while suppressing the hydrogenolysis of MCH. In terms of product distribution, the reaction products were n-heptane (n-C₇H₁₆ (see "n-C₇" in the table)), 3-ethylpentane (3-C₂H₅-C₅H₁₁ (see "3-EP" in the table)), 3-methylhexane (3-CH₃-C₆H₁₃ (see "3-MH" in the table)), 2-methylhexane (2 -CH₃-C₆H₁₃ (see "2-MH" in the table)), 3-methylpentane (3-CH₃-C₅H₁₁ (see "3-MP" in the table)), and 2-methylpentane (2-CH₃-C₅H₁₁ (see "2-MP" in the table)), as direct ring-opening products, light hydrocarbons (C₁ to C₅) as decomposition products, and toluene (TOL) as a dehydrogenation product.

The performance test results of the respective catalysts shown in Tables 3-5 above confirmed that the use of metal catalysts with iridium or rhodium supported on silica-based carriers such as amorphous silica or crystalline silicalite-1 allows highly selective hydrogenolysis of cyclopentanes included in methylcyclohexane.

The present invention has been described above based on specific embodiments, but these embodiments are merely examples, and the present invention is not limited by these embodiments. Not all the components of the metal catalyst and the method for hydrogenolysis of cyclic compounds using the same shown in the above-mentioned embodiments are essential, and at least those skilled in the art can select the components as appropriate within the scope of the present invention.

For example, application of the metal catalyst according to the present invention and the method for hydrogenolysis of cyclic compounds using the same is not limited to the hydrogenolysis device 6 provided in the hydrogen storage and transport system 1, but they are widely applicable for use in hydrogenolysis of cyclic compounds included in a saturated cyclic compound and having a five-membered structure.

As well as amorphous silica and MFI zeolites, mesoporous silica (e.g., SBA-15), for example, can be used as the silica-based carriers,.

### [Reference Signs List]

- 1: :transport system
- 2: :hydrogenation plant
- 3: :dehydrogenation plant
- 4: :storage facility
- 5: :storage facility
- 6: :hydrogenolysis device
- 11: :hydrogenation reaction device
- 12: :MCH storage tank
- 13: :TOL storage tank
- 21: :dehydrogenation reaction device
- 22: :TOL storage tank
- 23: :MCH storage tank

## Claims

1. A metal catalyst used in hydrogenolysis of a cyclic compound that has a five-membered ring structure and that is included in a saturated cyclic compound used as a hydrogen carrier, comprising:
a silica-based carrier as a catalyst carrier; and
iridium or rhodium as metal supported on the catalyst carrier.

2. The metal catalyst according to claim 1, wherein:
metal supported on the catalyst carrier is iridium; and
the metal catalyst includes 0.1 to 5 wt.% iridium as the metal.

3. The metal catalyst according to claim 1, wherein:
metal supported on the catalyst carrier is rhodium; and
the metal catalyst includes 0.5 to 2 wt.% rhodium as the metal.

4. The metal catalyst according to claim 1, wherein the silica-based carrier includes amorphous silica.

5. The metal catalyst according to claim 1, wherein the silica-based carrier includes MFI zeolite.

6. A method for hydrogenolysis, using the metal catalyst according to any one of claims 1 to 5, of a cyclic compound that has a five-membered ring structure and that is included in the saturated cyclic compound used as a hydrogen carrier, comprising:
producing an aromatic compound, and a cyclic compound that has a five-membered ring structure as an impurity, by dehydrogenation of the saturated cyclic compound;
performing hydrogenation of the aromatic compound with the impurity mixed therein; and
performing hydrogenolysis of mixture of the saturated cyclic compound and the impurity as produced in the hydrogenation of the aromatic compound, using the metal catalyst.

7. The method for hydrogenolysis according to claim 6, wherein the hydrogenolysis is performed by feeding the mixture of the saturated cyclic compound and the impurity to the metal catalyst with a catalyst layer thereof maintained at 150 °C to 300 °C together with 50 to 99.8 mol % hydrogen.
